# EUROPEAN PATENT SPECIFICATION

(11) **EP 3 377 007 B1**
(45) Date of publication and mention of the grant of the patent: **12.02.2020**
(21) Application number: 16798712.2
(22) Date of filing: 18.11.2016
(51) Int. Cl.: A61F 7/02, A42B 1/04

(54) **HEAD CAP FOR A COOLING PAD FOR USE IN A NON-INVASIVE MEDICAL COOLING PROCESS**
KOPFKAPPE FÜR EIN KÜHLKISSEN ZUR BENÜTZUNG IN NICHT-INVASIVEM MEDIZINISCHEN KÜHLPROZESS
CALOTTE DE TÊTE POUR COUSSIN DE REFROIDISSEMENT POUR L'USAGE DANS UN PROCÉDÉ DE REFROIDISSEMENT MÉDICAL NON-INVASIVE

(30) Priority: 19.11.2015 SE 1551500
(43) Date of publication of application: 26.09.2018
(73) Proprietor: Braincool AB, 223 81 Lund (SE)
(72) Inventor: BERG, Jon, 247 54 Dalby (SE)
(74) Representative: Ström & Gulliksson AB
(86) International application number: PCT/EP2016/078165
(87) International publication number: WO 2017/085273

(56) References cited:
- WO-A1-00/03666
- WO-A1-83/03527
- DE-A1-102005 050 970
- GB-A- 2 130 489
- US-A- 2 051 594
- US-A- 3 440 660
- US-A- 4 138 743

## Description

### TECHNICAL FIELD

The present invention relates to the field of non-invasive medical cooling of persons or patients, and in particular it relates to a head cap for a securing a medical cooling pad against the head of a person.

### BACKGROUND

Non-invasive medical cooling of parts or regions of the human body is a useful tool to reduce adverse impacts on the same under certain conditions. For example, medical cooling may be used for cooling the brain of patient suffering from a stroke. By reducing the temperature of the brain the risk of tissue damage is reduced for those parts of the brain lacking an adequate oxygen supply during the stroke. Another application of medical cooling is to cool the scalp for patients going through chemo therapy, to avoid undesirable hair loss.

There are different ways of reducing the body core temperature of a patient. For example, cooling pads may be applied to cool various regions of the patient body, e.g. to cool regions of the head or neck of a person who has suffered from stroke or a concussion of the brain. Depending on the application the cooling pads may be connected to a pump pumping cool liquid there through, whereby the heat from the body part to which the cooling pad is applied is heat exchanged away by means of the cooling pad. Cooling pads connected to a pump may typically be used in a healthcare environment, such as in an ambulance or at a hospital, where access to electric power is available to operate the pump.

A cooling pad typically consists of two superimposed sheets of flexible, fluid tight material that are sealed together along their edges. An inlet port is arranged in one end of the cooling pad for letting a flow of cooling fluid into the pad and an outlet port is provided in another end allowing the flow of cooling fluid out of the cooling pad. The inlet port and/or outlet port may be operatively connected to a pump for pumping the liquid through the cooling pad.

In other self sustainable (over a certain time period) cooling pads may be used. Such cooling pads may e.g. contain a very cool (relative to the normal body core temperature) medium such as ice or any other suitable medium. Such cooling pads may e.g. be used when the access to electrical power is limited or when a higher degree of mobility is required.

Regardless of the type of cooling pad, a general aim is to keep the rate of heat exchange between the patient and the cooling pad as high as possible. For a neck or head cooling application this may be realized by providing a head cap securing the cooling pad against the head of the user.

DE 10 2005 050970 A1 discloses the preamble of claim 1. Further prior art can be found in WO00/03666, US patent no. 2,051,594, WO83/03527 A1, US patent no. 3,440,660, GB2130489 and US patent no. 4,138,743. However, there is a need of providing a head cap which improves the overall management and user friendliness of the medical cooling process. Moreover, a head cap improving the way the cooling pad secured to the head and/or neck of the user would also be advantageous.

### SUMMARY

The head cap according to the invention is defined by the appended independent claim. Preferred embodiments are listed in the dependent claims. The head cap according to the invention provides for an improved overall management and user friendliness of the medical cooling process. More specifically, the head cap of both aspects is easier to mount on the patients head. Hence, the mounting process of the herein provided head cap takes is considerably more time efficient, which means that the medical cooling process may be initiated quicker than is possible with conventional head caps. Moreover, the head cap of both aspects allows for improved securing to the head of the person.

### BRIEF DESCRIPTION OF THE DRAWINGS

In order to explain the present invention the associated head cap will be described with reference to the drawings, in which:
Fig. 1 is a perspective front view of a head cap mounted on the head of a patient where the closeable slit is in its closed position, the neck straps are shown in a closed mutually attached position, and the head straps are shown in a first attached position;
Fig. 2 is a perspective side view of the head cap of Fig. 1;
Fig. 3 is a perspective rear view of the head cap of Fig. 1;
Figs 4 and 5 respectively show the head cap in an open position;
Fig. 6 shows the head cap in an open position when the head of a patient is placed therein;
Fig. 7 is a perspective front view of a head cap mounted on the head of a patient where the closeable slit is in its closed position while the neck straps are shown in a open non-attached position;
Fig. 8 is a perspective front view of a head cap mounted on the head of a patient where the closeable slit is in its open position while closed while the neck straps are shown in a closed mutually attached position;
Fig. 9 is a perspective front view of a head cap mounted on the head of a patient where the closeable slit is in its closed position and the neck straps are shown in a closed mutually attached position while the head straps are shown in a non-attached open position;
Fig. 10 is a perspective rear view of the head cap of Fig. 9;
Fig. 11 is a perspective front view of a head cap mounted on the head of a patient where the closeable slit is in its closed position, the neck straps are shown in a closed mutually attached position while the head straps are shown in a second attached position; and
Fig. 12 is a perspective side view of the head cap of Fig. 11.

### DETAILED DESCRIPTION

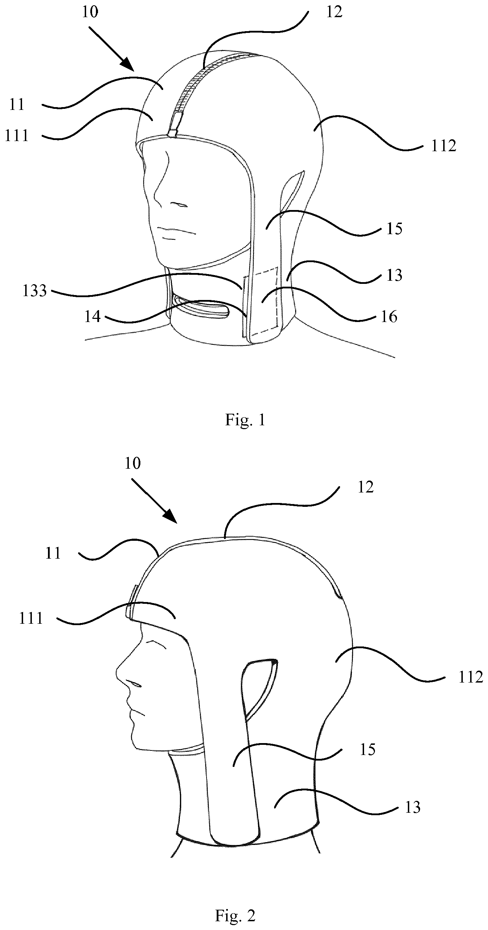

Fig. 1 is a perspective front view showing a head cap 10 for securing a medical cooling pad against the head of a patient in use. The head cap comprises a head portion 11 for surrounding the scalp of the patient. The head portion 11 comprises a front part 111 provided with a closeable slit 12. The closeable slit extends from a front outermost edge in a direction towards a rear part 112 of the head portion 11. The direction has a component in the sagittal, e.g. median, plane with reference to the intended position of the head of the patient in use. The definition of the sagittal or median plane in terms of the head cap hence implies a plane which divides the head cap in two symmetrically identical mirrored halves. In some embodiments, the extension of the slit may be arranged parallel to the sagittal plane as shown throughout the drawings, however it may also be provided at an angle to the defined sagittal plane. The head cap 10 further comprises a neck portion 13 for surrounding the neck of the patient in use. The neck portion is connected to the rear part 112 of the head cap. At each exterior lateral side 133 the neck portion 13 comprises a first attachment member 14. Two head straps 15 are connected to either lateral side of the front part 111. Each head strap 15 comprises a second attachment member 16 for attachment to the respective first attachment member 14.

With reference to Fig. 1 is may be observed that the closeable slit is provided with a zipper. Although a zipper may be preferred as it is very easy to handle, any other suitable known means of providing the slit with an opening and closing mechanism, e.g. a zip lock, could also be used without departing from the scope of the claims.

The first 14 and second 16 attachment members may be made of a Velcro material for providing a mutual attachment there between. Although a Velcro solution may be preferred under some conditions, it should be appreciated that any suitable way of attaching the head strap to the exterior lateral side 133 of the neck portion 13 could be used without departing from the scope of the claims.

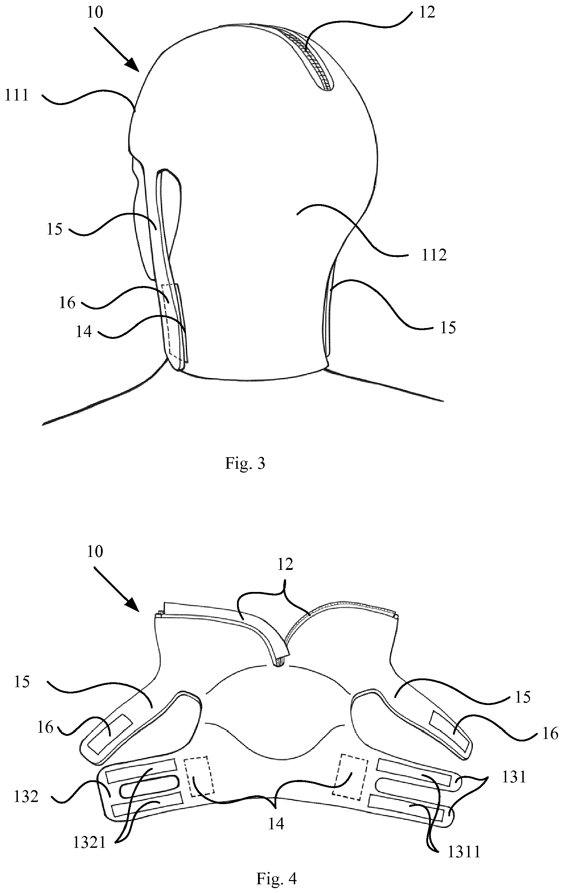

Corresponding features to those shown in Fig. 1 may also be found in Fig. 2 which is a perspective side view of the head cap of Fig. 1 as well as Fig. 3 which is a perspective rear view of the head cap of Fig. 1.

As perhaps is best shown with reference to Figs 4 to 6, the neck portion 13 comprises a first neck strap 131 provided with a third attachment member 1311. The neck portion 13 further comprises a second neck strap 132 provided with a fourth attachment member 1321 for attachment to the third attachment member 1311 in use. By attaching the third and fourth attachment members the neck portion may be secured around the patient's neck, thereby encompassing the neck of the patient.

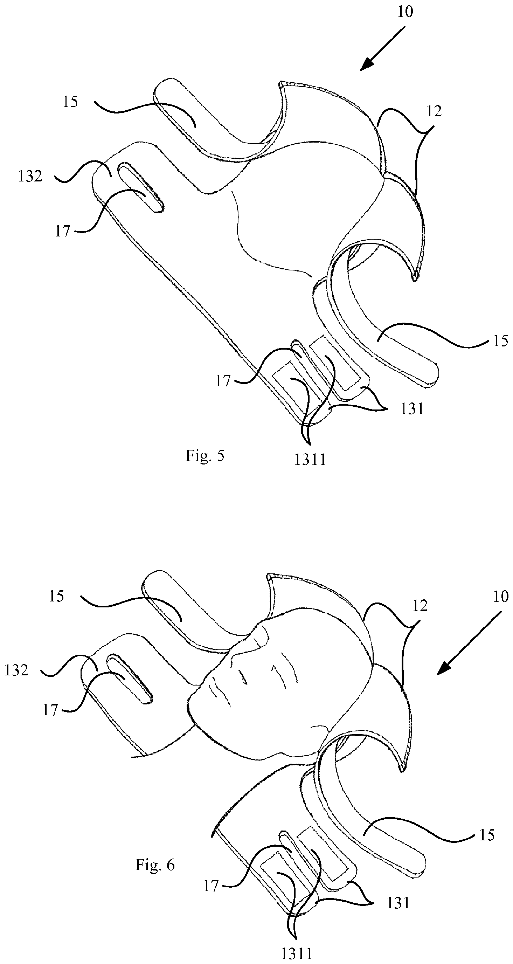

Fig. 4 and 5 respectively show the head cap in a fully open position, whereas Fig. 6 shows the head cap in its fully open position when the head of a patient is placed therein.

In the fully open position of the head cap, the closeable slit is in its open position thereby allowing the two lateral side parts of the front part 111 to fold apart and attaining an essentially planar shape. Moreover, in the fully open position the respective first attachment members provided on the head straps 15 are not attached to the respective second attachment members and are thus free to move. Moreover, the first and second neck straps are not attached to each other in the fully open position of the head cap.

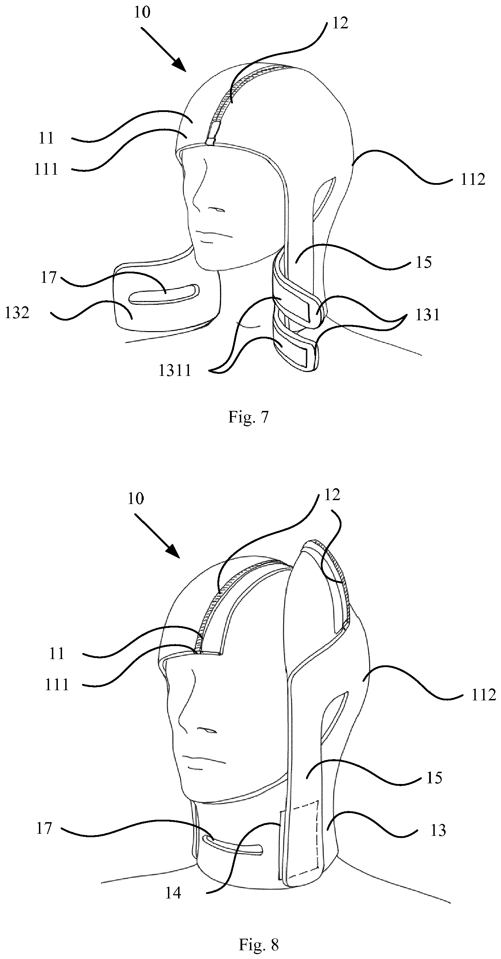

Figs 4 to 7 moreover show one way of mounting the head cap onto the patient's head. For example, with reference to Fig. 4 the head cap is placed on a flat surface, such as a foundation, next to the patient. In the fully open position the head cap attains a near flat shape. Fig. 5 may be said to show a state before the head cap is placed on the flat surface, i.e. before the head cap attains the shape shown in Fig. 4. Once, the head cap is placed on the flat surface the patient's head may be gently onto the fully opened head cap as shown in Fig. 6. Alternatively, in the event the patient is lying on the ground, the patient's head may be gently lifted to make enough room for the fully opened head cap to be put in position between the patient's head and the underlying foundation. Once, the patient's head is positioned on the head cap cooling pads (not shown in the drawings) may provided between the patient's head and the interior surface of the head cap. Next the closeable slit 12 may be closed as shown with reference to Fig. 7 followed by attaching the first and second neck straps to each other around the neck of the patient. Optionally, as shown with reference to Fig. 8, the first 131 and second 132 neck straps may be mutually attached before closing the closeable slit 12. Also the respective head straps 15 may be attached in any order such as to secure the cooling pad towards the head of the patient.

Similarly to that of the first and second attachment members, the third 1311 and fourth 1321 attachment members may be made of a Velcro material, however other suitable materials could also be used.

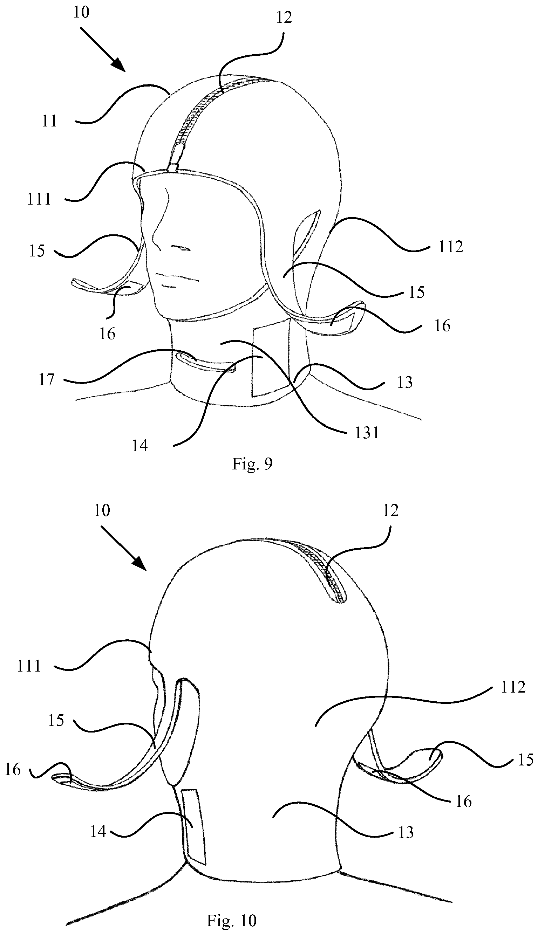

As perhaps best seen with reference to Fig. 9, at least one part of the first neck strap 131 and second neck strap 132 forms the exterior lateral side of the neck portion 13 at which the first attachment member 14 is arranged. The first attachment member 14 may be provided on an exterior side of the respective neck straps 131, 132.

As seen with reference to Figs 5 to 9, at least one of the first 131 or second 132 neck strap may comprise an opening 17 for receiving the laryngeal prominence of the patient. In this way there will be no pressure from the respective neck strap against the Adam's apple which drastically increases the comfort for the patient.

The third attachment member 1311 may be attached to an interior side of the first neck strap 131 while the fourth attachment member 1321 is attached to an exterior side of the second neck strap or vice versa. For example, with reference to Fig. 5 the third attachment member 1311 is provided on an interior side (i.e. the side facing the patient's neck in use when the two neck straps are attached), whereas the fourth attachment member 1321 is provided on an exterior side of the second neck strap 132 (not shown since the interior side of the second neck strap covers the exterior side in Fig. 5).

Additionally or alternatively, attachment members 1311,1321 may be provided on both the interior side and exterior side of each neck strap in order to increase the variation of attachment possibilities, allowing for more user friendliness for the user or personnel mounting the head cap on the patient's head.

Each second attachment member 16 is provided at an interior side, i.e. the side facing the patient's head in use, of the associated head strap 15. Such a configuration is shown e.g. in Figs 1 through 4 and allow for the exterior surface of the head cap to closely follow the contour of the patient's head when the second attachment member 16 attaches to the first attachment member provided at the exterior lateral side 133 of the neck portion 13.

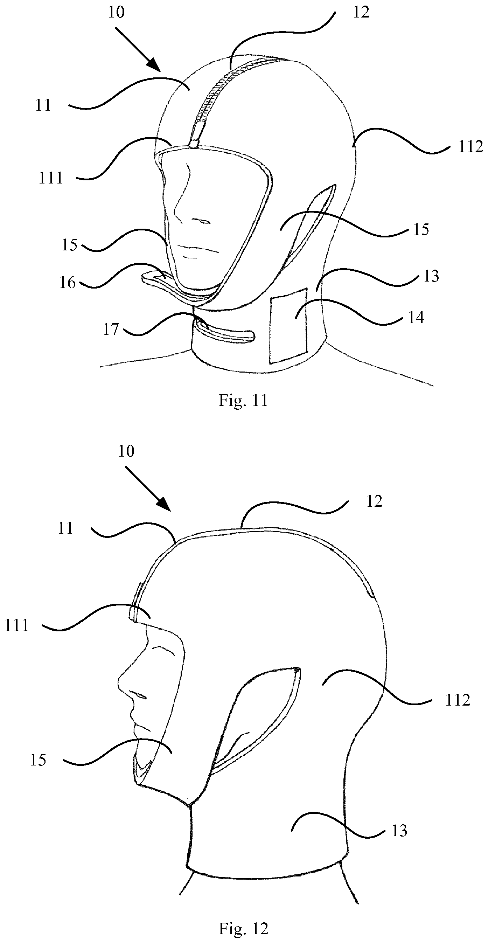

According to the invention, there is provided a second attachment member 16 on both an interior side and an exterior side of at least one of the head straps 15. This allows for the possibility of attaching a second attachment member 16 of a first head strap 15 to a further second attachment member 16 provided on the other head strap 15, e.g. below the chin of the patient. Such a configuration is shown with reference to Figs 11 and 12. The ability of attaching each head strap at different positions of the head cap increases the flexibility of the device. It should be noted that while the configuration of Figs 11 and 12 are optional, a preferred position of attaching the head straps is at the exterior lateral side 133 of the neck portion 13 as shown with reference to Figs 1 to 3 and 7 to 8. The reason being that the direction of the force by the head cap onto the cooling pad and head of the patient when attached to the exterior lateral side of the patient's neck, is more perpendicular to the head surface at the front part 111 of the head cap. This means that the force or pressure of the head cap towards the head of the patient will be improved in the front part 111 of the head cap, when attaching the head straps to the exterior lateral side 133 of the neck portion 13. Another benefit of attaching the head straps 15 to the exterior lateral side of the neck portion 13 is that the patient's mouth will not be forced closed, which allows health care staff to access the mouth, e.g. to clear the airways by using a laryngoscope while the patient undergoes medical cooling.

The head cap may be made of a suitable elastic or flexible material. Overall, an elastic material of the whole head cap is preferred while it is possible to provide only some parts of the head cap, such as the head straps or neck straps in such a material.

One particularly, suitable elastic material is neoprene material. The present inventors have found that the neoprene material, when the cooling pad is made from silicon, provides for a superior friction between the two components in use.

## Claims

1. A head cap (10) for securing a cooling pad against the head of a person in use, comprising
a head portion (11) for surrounding the scalp of the person, the head portion comprising a front part (111),
a neck portion (13) for surrounding the neck of the person in use and connected to a rear part (112) of the head cap, wherein the neck portion (13) at each exterior lateral side (133) thereof comprises a first attachment member (14), and wherein
the front part (111) at each lateral side thereof comprises a head strap (15) provided with a second attachment member (16) at an interior side thereof for attachment to the respective first attachment member (14), **characterized in that** at least one of the head straps (15) comprises one further second attachment member (16) provided at an exterior side thereof, so as to allow for attaching said at least one head strap (15) to the second attachment member (16) of the other head strap (15) below the chin of the patient, **and that** the front part (111) is provided with a closeable slit (12) extending from a front outermost edge in a direction having a component in a median plane towards the rear part (112) of the head portion (11), such that the head cap in a fully open position, when the closeable slit (12) is in its open position, attains a near flat shape.

2. The head cap (10) according to claim 1, wherein the closeable slit (12) is provided with a zipper or zip lock.

3. The head cap (10) according to claim 1 or 2, wherein the first and second attachment members (14, 16) are made of Velcro material.

4. The head cap (10) according to any of the claims 1 to 3, wherein the neck portion (13) further comprises
a first neck strap (131) provided with a third attachment member (1311),
a second neck strap (132) provided with a fourth attachment member (1321) for attachment to the third attachment member (1311) in use for encompassing the neck of the person.

5. The head cap according to claim 4, wherein the third and fourth attachment members are made of Velcro material.

6. The head cap according to claim 4 or 5, wherein at least one part of the first neck strap (131) and second neck strap (132) forms the exterior lateral side of the neck portion (13) at which the first attachment member (14) is arranged.

7. The head cap according to any of claims 4 to 6, wherein at least one of the first (131) or second (132) neck strap comprises an opening (17) for receiving the laryngeal prominence of the person.

8. The head cap according to any of claims 4 to 7, wherein the third attachment member (1311) is attached to an interior side of the first neck strap (131) while the fourth attachment member (1321) is attached to an exterior side of the second neck strap (132), or wherein the third attachment member (1311) is attached to an exterior side of the first neck strap (131) while the fourth attachment member (1321) is attached to an interior side of the second neck strap (132).

9. The head cap according to any of the preceding claims, wherein each second attachment member (16) is provided at an interior side of the associated head strap (15).

10. The head cap according to any of the preceding claims, wherein at least one head strap (15) comprises one second attachment member (16) provided at an interior side thereof and a further second attachment member (16) provided at an exterior side thereof.

11. The head cap according to any of the preceding claims, being made of a flexible material.

12. The head cap according to any of the preceding claims, being made of a neoprene material.

## Patentansprüche

1. Kopfkappe (10) zur Sicherung eines Kühlkissens am Kopf einer verwendenden Person, umfassend
einen Kopfabschnitt (11) zum Umgeben der Kopfhaut der Person, wobei der Kopfabschnitt ein Vorderteil (111) umfasst,
einen Halsabschnitt (13) zum Umgeben des Halses der verwendenden Person, der mit einem Hinterteil (112) der Kopfkappe verbunden ist,
wobei der Halsabschnitt (13) an jeder seitlichen Außenseite (133) desselben ein erstes Befestigungselement (14) umfasst, und wobei
das Vorderteil (11) an jeder seitlichen Außenseite desselben ein Kopfband (15) umfasst, das mit einem zweiten Befestigungselement (16) an einer Innenseite desselben zur Befestigung an dem entsprechenden ersten Befestigungselement (14) versehen ist, **dadurch gekennzeichnet, dass** mindestens eines der Kopfbänder (15) ein weiteres zweites Befestigungselement (16) umfasst, das an einer Außenseite desselben vorgesehen ist, um das Befestigen dieses mindestens einen Kopfbands (15) an dem zweiten Befestigungselement (16) des anderen Kopfbands (15) unterhalb des Kinns der verwendenden Person zu ermöglichen, **und dass** das Vorderteil (111) mit einem verschließbaren Schlitz (12) versehen ist, der sich von einer äußersten Vorderkante in eine Richtung mit einer Komponente in einer Medianebene in Richtung des Hinterteils (112) des Kopfabschnitts (11) erstreckt, so dass die Kopfkappe in einer vollständig geöffneten Position, wenn der verschließbare Schlitz (12) in seiner geöffneten Position ist, eine nahezu flache Form erreicht.

2. Kopfkappe (10) nach Anspruch 1, wobei der verschließbare Schlitz (12) mit einem Reißverschluss oder Zippverschluss versehen ist.

3. Kopfkappe (10) nach Anspruch 1 oder 2, wobei das erste und zweite Befestigungselement (14, 16) aus Klettband bestehen.

4. Kopfkappe (10) nach irgendeinem der Ansprüche 1 bis 3, wobei der Halsanschnitt (13) ferner umfasst
ein ersten Halsband (131), das mit einem dritten Befestigungselement (1311) versehen ist,
ein zweites Halsband (132), das mit einem vierten Befestigungselement (1321) zur Befestigung an dem verwendeten dritten Befestigungselement (1311) versehen ist, um den Hals der Person zu umschließen.

5. Kopfkappe nach Anspruch 4, wobei das dritte und vierte Befestigungselement aus Klettband bestehen.

6. Kopfkappe nach Anspruch 4 oder 5, wobei mindestens ein Teil des ersten Halsbands (131) und des zweiten Halsbands (132) die seitliche Außenseite des Halsabschnitts (13) bildet, an dem das erste Befestigungselement (14) angeordnet ist.

7. Kopfkappe nach irgendeinem der Ansprüche 4 bis 7, wobei mindestens eines des ersten (131) oder zweiten (132) Halsbands eine Öffnung (17) zur Aufnahme der Prominentia laryngea einer Person umfasst.

8. Kopfkappe nach irgendeinem der Ansprüche 4 bis 7, wobei das dritte Befestigungselement (1311) an einer Innenseite des ersten Halsbands (131) befestigt ist, während das vierte Befestigungselement (1321) an einer Außenseite des zweiten Halsbands (132) befestigt ist, oder dass das dritte Befestigungselement (1311) an einer Außenseite des ersten Halsbands (131) befestigt ist, während das vierte Befestigungselement (1321) an einer Innenseite des zweiten Halsbands (132) befestigt ist.

9. Kopfkappe nach irgendeinem der vorhergehenden Ansprüche, wobei jedes zweite Befestigungselement (16) an einer Innenseite des zugehörigen Kopfbands (15) vorgesehen ist.

10. Kopfkappe nach irgendeinem der vorhergehenden Ansprüche, wobei mindestens ein Kopfband (15) ein zweites Befestigungselement (16), das an einer Innenseite desselben vorgesehen ist, und ein weiteres zweites Befestigungselement (16), das an einer Außenseite desselben vorgesehen ist, umfasst.

11. Kopfkappe nach irgendeinem der vorhergehenden Ansprüche, wobei sie aus einem flexiblen Material besteht.

12. Kopfkappe nach irgendeinem der vorhergehenden Ansprüche, wobei sie aus einem Neoprenmaterial besteht.

## Revendications

1. Calotte de tête (10) pour retenir, en cours d'utilisation, un coussin de refroidissement contre la tête d'une personne, comprenant
une portion de tête (11) pour entourer le cuir chevelu de la personne, la portion de tête comprenant une partie avant (111),
une portion de cou (13) pour entourer, en cours d'utilisation, le cou de la personne et reliée à une partie arrière (112) de la calotte de tête,
dans laquelle la portion de cou (13) sur chaque côté latéral extérieur (133) de celle-ci comprend un premier élément de fixation (14), et dans laquelle
la partie avant (111) sur chaque côté latéral de celle-ci comprend une sangle de tête (15) pourvue d'un deuxième élément de fixation (16) sur un côté intérieur de celle-ci pour se fixer au premier élément de fixation (14) respectif, **caractérisée en ce qu'**au moins l'une des sangles de tête (15) comprend un deuxième élément de fixation (16) supplémentaire prévu sur un côté extérieur de celle-ci, de sorte à permettre la fixation de ladite au moins une sangle de tête (15) au deuxième élément de fixation (16) de l'autre sangle de tête (15) sous le menton du patient, et **en ce que** la partie avant (111) est pourvue d'une fente fermante (12) s'étendant depuis un bord avant le plus à l'extérieur dans une direction ayant une composante dans un plan médian vers la partie arrière (112) de la portion de tête (11), de sorte que la calotte de tête en position entièrement ouverte, lorsque la fente fermante (12) est dans sa position ouverte, prenne une forme presque plate.

2. Calotte de tête (10) selon la revendication 1, dans laquelle la fente fermante (12) est pourvue d'une glissière ou d'une fermeture à glissière.

3. Calotte de tête (10) selon la revendication 1 ou 2, dans laquelle les premier et deuxième éléments de fixation (14, 16) sont réalisés en matériau en ruban auto-agrippant.

4. Calotte de tête (10) selon l'une quelconque des revendications 1 à 3, dans laquelle la portion de cou (13) comprend en outre
une première sangle de cou (131) pourvue d'un troisième élément de fixation (1311),
une deuxième sangle de cou (132) pourvue d'un quatrième élément de fixation (1321) pour se fixer, en cours d'utilisation, au troisième élément de fixation (1311) pour englober le cou de la personne.

5. Calotte de tête selon la revendication 4, dans laquelle les troisième et quatrième éléments de fixation sont réalisés en matériau en ruban auto-agrippant.

6. Calotte de tête selon la revendication 4 ou 5, dans laquelle au moins une partie de la première sangle de cou (131) et de la deuxième sangle de cou (132) forme le côté latéral extérieur de la portion de cou (13) sur laquelle le premier élément de fixation (14) est agencé.

7. Calotte de tête selon l'une quelconque des revendications 4 à 6, dans laquelle au moins l'une parmi la première (131) ou la deuxième (132) sangle de cou comprend une ouverture (17) pour recevoir la proéminence du larynx de la personne.

8. Calotte de tête selon l'une quelconque des revendications 4 à 7, dans laquelle le troisième élément de fixation (1311) est fixé sur un côté intérieur de la première sangle de cou (131) tandis que le quatrième élément de fixation (1321) est fixé sur un côté extérieur de la deuxième sangle de cou (132), ou dans laquelle le troisième élément de fixation (1311) est fixé sur un côté extérieur de la première sangle de cou (131) tandis que le quatrième élément de fixation (1321) est fixé sur un côté intérieur de la deuxième sangle de cou (132).

9. Calotte de tête selon l'une quelconque des revendications précédentes, dans laquelle chaque deuxième élément de fixation (16) est prévu sur un côté intérieur de la sangle de tête (15) associée.

10. Calotte de tête selon l'une quelconque des revendications précédentes, dans laquelle au moins une sangle de tête (15) comprend un deuxième élément de fixation (16) prévu sur un côté intérieur de celle-ci et un deuxième élément de fixation (16) supplémentaire prévu sur un côté extérieur de celle-ci.

11. Calotte de tête selon l'une quelconque des revendications précédentes, réalisée en un matériau flexible.

12. Calotte de tête selon l'une quelconque des revendications précédentes, réalisée en un matériau néoprène.
